# EUROPEAN PATENT APPLICATION

(11) **EP 1 209 226 A2**
(43) Date of publication of application: **29.05.2002**
(21) Application number: 01125374.7
(22) Date of filing: 30.10.2001
(51) Int. Cl.: C12N 5/06, C12N 5/08, C07K 14/47, A61K 38/17, A61K 35/14, A61P 35/00

(54) **Maturation of dendritic cells by recombinant heat shock protein 70 (hsp70)**

(30) Priority: 07.11.2000 DE 10055213; 29.03.2001 DE 10115439
(71) Applicant: GSF-Forschungszentrum für Umwelt und Gesundheit GmbH, 85764 Oberschleissheim (DE)
(72) Inventor: Kuppner, Maria, Dr., 82151 Planegg (DE); Issels, Rolf D., Dr., 80798 München (DE); Gastpar, Robert, Dr., 81241 München (DE)
(74) Representative: Reinhard - Skuhra - Weise & Partner

(57) **Abstract**

This invention relates to an ex vivo method for inducing the TNF-α free differentiation of immature dendritic cells into mature dendritic cells as well as an ex vivo method for generating said mature dendritic cells. The induction of the differentiation of immature dendritic cells into mature dendritic cells is accomplished by using an effective amount of heat shock proteins of the hsp70 family or a biologically active part thereof. The invention further relates to mature dendritic cells otainable by said ex vivo method. Furthermore, the invention is directed to therapeutic compositions comprising an effective amount of heat shock proteins of the hsp70 family or of said mature dendritic cells as well as their use in the immunotherapy of neoplastic diseases.

## Description

This invention relates to ex vivo methods for inducing the TNF-α free differentiation of immature dendritic cells into mature dendritic cells as well as ex vivo methods for generating said mature dendritic cells. The invention further relates to mature dendritic cells obtainable by said ex vivo method. Furthermore, the invention is directed to therapeutic compositions comprising an effective amount of heat shock proteins of the hsp70 family or of said mature dendritic cells as well as their use in the immunotherapy of neoplastic diseases.

Cells respond to stress factors such as heat, hypoxia or viral transformation by the synthesis of a group of proteins called heat shock proteins (hsp) [1]. Members of the hsp70 group are either constitutively expressed (hsc70) or can be induced (hsp70) by stress factors [2]. They function as molecular chaperones for antigenic peptides in the endoplasmic reticulum and cytoplasm and are involved in antigen processing and presentation [3]. As previously shown by the inventors, Hsp70 is also expressed on the surface of human tumor cells such as sarcomas, lung carcinoma and colon carcinoma [4-7] and can act as a recognition structure for NK cells [8]. It has been proposed that hsps can also activate the innate immune response by acting as danger signals [9-10] since hsp70 [11] and hsp60 [9] can directly induce the production of cytokines from monocytes and macrophages. Danger signals [12] are thought to be recognized by pattern recognition receptors on antigen presenting cells (APCs) [13-14]. Professional APCs such as dendritic cells initiate an immune response after activation and form the link between the innate and an acquired immune response [15]. Immature DC specialize in antigen capture and processing whereas mature DC are potent antigen presenting cells [17]. Dendritic cells are now widely recognized to play an important role in the immune response to tumors [16]. Therefore, the maturation of dendritic cells, i.e. the differentiation from immature into mature dendritic cells, has been a subject of intense investigation in the last years.

Recently it has been shown by Singh-Jasuja et al.(2000) that the constitutively expressed hsp gp96 can induce the maturation of dendritic cells derived from CD14+ monocytes [18].

However, high concentrations of gp96 (in the range of 30 - 100 µg/ml protein) were needed for the generation of mature dendritic cells. An enhanced ability of the mature dendritic cells to present peptides to specific T cells has not been described.

Moreover, there have been further approaches in the prior art for inducing the differentiation of monocytes to mature dendritic cells:

US Patent No. 5 849 589 (published on December 12, 1998) describes a method for inducing the differentiation of a population of monocytes into a population of cells comprising greater than 50% mature CD83@+ dendritic cells, said method comprising culturing monocytes in an induction medium comprising granulocyte/ macrophage-colony stimulating factor ("GM-CSF"), interleukin-4 ("IL-4"), and tumor necrosis factor-alpha ("TNF-alpha"), said GM-CSF, IL-4, and TNF-alpha being present simultaneously in said induction medium in sufficient amounts to induce said differentiation.

French Patent No. 2 777 906 (published on October 29, 1999) describes a process for obtaining human dendritic cells from monocytes in the presence of interleukin-4 (IL-4), granulocyte macrophage colony stimulating factor (GM-CSF) and tumor necrosis factor alpha (TNF-alpha). The process comprises several crucial parameters comprising: (a) the addition of 1-3 mg/ml bicarbonate to the culture medium; (b) maintaining the pH at 7.2-7.4; (c) culturing in Teflon pots; and (d) using 1-25% autologous/homologous human serum.

The above mentioned methods are suffering from the severe drawbacks that are related to the use of TNF-α. TNF-α has a preeminent role in initiating the immune response. While it is normally beneficial to the host, in situations of over-production, TNF-α itself can kill the host. For example, excess acute levels of TNF-α have been associated with toxic shock syndrome, while chronic over-production is associated with inflammatory bowel disease, rheumatoid arthritis, and cirrhosis of the liver.

In the therapy, the dose-limiting toxicity of TNF-α consists of thrombocytopenia, headache, confusion and hypotension. Thus, the toxicity of systemically administered TNF-α seriously limits its use for therapeutic purposes. TNF-α has been most effective when used for regional therapy, in which measures, such as limb isolation for perfusion, are taken to limit the systemic dose and hence the toxicity of TNF-α (Mittleman, A., et al., 1992, Inv. New Drugs 10:183-190).

Therefore, TNF-α containing compositions are not suitable for preparations which are intended for use, for example, in the clinical immunotherapy.

Therefore, it is an object of the present invention to provide methods for the maturation of dendritic cells which avoid the use of TNF-α. It is a further object of the present invention to provide mature dendritic cells, which show an enhanced ability to present peptides to specific T cells.

These objects are solved by the features of the independent claims. Preferred embodiments are set out in the dependent claims.

By using an effective amount of heat shock proteins of the hsp70 family or a biologically active part thereof, an induction of the maturation of immature dendritic cells (DC) derived from monocyte precursors to mature dendritic cells (DC) is performed. Furthermore, immature DC stimulated to mature DC with hsp70, for example with recombinant hsp70 (rhsp70, definition see below), show an enhanced ability to present peptides to specific CTL (cytotoxic T-lymphocytes) . Already small amounts of hsp70 showed a higher effectiveness in inducing immature dendritic cells than previously used TNF-α. Therefore, hsp70 is useful for its adjuvant like properties in DC based immunotherapy of certain tumors and may be used as an alternative for the toxic cytokine TNF-α in the maturation of DC's.

In general, the invention describes an ex vivo method for inducing the TNF-α free differentiation of immature dendritic cells into mature dendritic cells, said method comprising contacting the immature dendritic cells with an effective amount of heat shock proteins of the hsp70 family or a biologically active part thereof.

Additionally, the invention comprises an in vivo method for generating mature dendritic cells, by performing the steps of inducing the differentiation of immature dendritic cells into mature dendritic cells, by contacting the immature dendritic cells with an effective amount of heat shock proteins of the hsp70 family or a biologically active part thereof free of TNF-α; and recovering said mature dendritic cells. Preferably, the recovery of the population of dendritic cells includes flow cytometry or cell isolation methods.

In another aspect, the biologically active part of the hsp70 protein is defined as the C-terminal domain of hsp 70. The hsp70s contain two principal domains. The N-terminal ATPase domain is the most conserved (about 64 % residue identity among eucaryotic Hsp70s) while the C-terminal part is occupied by a more variable peptide-binding domain. This C-terminal domain turned out to show biological activity in the maturation of dendritic cells.

According to one preferred embodiment, the immature dendritic cells are generated by culturing monocytes in an induction medium containing granulocyte/macrophage-colony stimulating factor ("GM-CSF") and interleukin-4 ("IL-4"). Said cytokine mixture gives optimal results, although other mixtures have to be considered. Therefore, the invention is not limited to the use of said cytokines. It is within the general knowledge of the skilled artisan to use other cytokine mixtures, where appropriate. Examples of other cytokines are IFN-α, IFN-γ, IL1-β, IL-2, PGE2 or IL-6.

The concentrations of GM-CSF and IL-4 range from 125 to 2000 U/ml. Preferably, the concentrations of GM-CSF and IL-4 are between 500-1000 U/ml.

In a preferred embodiment, the monocytes are plastic-adherent human blood monocytes and the dendritic cells therefore are human dendritic cells which are capable of inducing T cell proliferation.

According to one preferred embodiment, a method is provided, in which the dendritic cells are pulsed with an antigenic agent subsequent to maturation. One example of such an antigenic agent is the tyrosinase 369-377 nonapeptide. Other preferred examples of antigenic agents are tumor derived peptides and viral antigenic peptides.

According to a preferred embodiment, the hsp70 used is recombinant hsp70 (rhsp70). This rhsp70 may, for example, be generated by inserting the human gene for hsp70 into bacteria, which would then express the protein. Bacterial products may be removed by binding to ATP agarose.

The hsp70 proteins can be used in a broad range of concentrations. However, in a preferred embodiment, the method involves maintaining the rhsp70 concentration in the medium in the range of about 0.1 - 1.0 µg/ml and more preferably 0.5 µg/ml.

The invention is further directed to a TNF-α free therapeutic composition for inducing the maturation of immature dendritic cells comprising, as the only active maturation agent, an effective amount of heat shock proteins of the hsp70 family, preferably rhsp70, or a biologically active part thereof, in combination with a pharmaceutically acceptable carrier.

In another embodiment, the invention is directed to a therapeutic composition comprising the mature dendritic cells made according to the methods of the present invention, in combination with a pharmaceutically acceptable carrier.

These compositions preferably are vaccines, in which the hsp70/dendritic cells are present in physiological saline, suitable for administration by injection. For example, such a vaccine may be used by administering the dendritic cell containing composition to a mammal, preferably to a human patient, for ex vivo cell transplantation therapy.

Furthermore, in one embodiment, the above mentioned therapeutic compositions are used in the immunotherapy of neoplastic diseases. These neoplastic diseases consist of, but are not limited to, solid tumors and leukemias, although the use in the therapy of solid tumors is preferred. Solid tumors comprise, for example, tumors associated with malignant melanoma, breast carcinoma, colon carcinoma, pancreas carcinoma, prostate carcinoma, ovarian carcinoma, mesothelioma, neuroblastoma, renal cell carcinoma, non-small cell lung carcinoma, and AIDS-associated Kaposi's sarcoma.

### Description of the drawings:

### Figure 1

FACS analysis of CD40, CD86 and CD83 expression in monocyte derived DC. Monocytes were cultured for 8 days in medium containing GM-CSF and IL-4 alone (grey histogram) or in GM-CSF/IL-4 plus rhsp70 (0.5µg/ml) added to the cultures on day 5 (black line, upper panel) or in GM-CSF/IL-4 plus rhsp70 (0.5µg/ml) heat denatured (100°C, 20 min) (black line, lower panel) added to the cultures on day 5. The dotted line represents isotype control antibodies which showed the same fluoresence intensities with the addition of rhsp70 or heat denatured rhsp70. The results are representative of 3 separate experiments.

### Figure 2

Comparison of the effect of rhsc 70 and rhp70 on DC maturation. A) CD83 and B) CD14 expression on DC cultured for 8 days in medium containing GM-CSF and IL-4 alone (white bar) or in GM-CSF/IL-4 plus rhsc70 (grey bar) added on day 5 or in GM-CSF/IL-4 plus increasing concentrations of rhsp70 (black bars) added on day 5. The results are representative of 2 separate experiments.

### Figure 3

Effect of polymyxin B on hsp70 induced DC maturation. DC were cultured for 8 days in GM-CSF and IL-4 containing medium alone (white bar) or with the rhsp70 (black bar) added on day 5 or with rhsp70 plus polymyxin B (0.5µg/ml) added on day 5 (grey bar). A FACS analysis of CD40, CD86, HLA-DR and CD83 expression was done on day 8. The results are representative of 2 separate experiments.

### Figure 4

Antigen specific T cell stimulation. DC were cultured for 8 days in medium containing GM-CSF and IL-4 alone (DC-hsp) or with rhsp70 (0.5µg/ml) added on day 5 of culture (DC+hsp). DC were then plated at 10⁴ / well in 100µl medium in 96 well round bottomed plates and pulsed with tyrosinase peptide 1µg/ml (white bar) or 10 µg/ml (black bar) for 2 h and then irradiated. 2x 10⁴ tyrosinase specific CTL were then added in a final volume of 100 µl to each well in medium containing 20% FCS and 100U/ml IL2. Control cultures containing non- tyrosinase pulsed DC plus CTL (dark grey bars) or DC alone (light grey bars) or CTL alone (CTL) were also included.

A. The cells were cultured for 72h and ³H thymidine was added for the last 24h of culture. The results are the mean values of triplicate cultures plus or minus the standard deviation. The results are representative of 3 separate experiments.

B. Cultures were set up in parallel for IFN-gamma production. Supernatants (100µl) were removed from each well after 24h and assayed for IFN-gamma production using an ELISA kit specific for IFN-gamma. The results are the mean values of triplicate cultures plus or minus the standard deviation. The results are representative of 3 separate experiments.

### Figure 5A.

Phenotypic analysis of monocyte derived DC cultured for 8 days in medium containing GM-CSF and IL-4 alone (grey histogram) or in GM-CSF/IL-4 plus rhsp70 (0.5µg/ml) (black line). The dotted line represents an isotype control antibody. The isotype controls showed similar levels of fluorescence for all the markers studied. The results are representative of 3 separate experiments.

### Figure 5B

CD83 expression in monocyte derived DC cultured for 8 days in medium containing GM-CSF and IL-4 alone (black histogram) or in GM-CSF/IL-4 plus increasing concentrations of rhsp70 (0.1-0.7 µg/ml) (grey histogram). The dotted line represents the isotype control fluorescence. The results are representative of 3 separate experiments.

### Figure 6

CD83 expression in monocyte derived DC cultured for 8 days in medium containing GM-CSF and IL4 alone (white bar) or in medium containing rhsp70 (0.5µg/ml) (black bar).

### Figure 7

CD40, CD86 and CD83 expression in monocyte derived DC cultured for 8 days in medium containing GM-CSF and IL-4 alone (white bar) or in GM-CSF and IL-4 plus rhsc70 (0.5) µg/ml (light grey bar) or rhsp70 (0.5µg/ml) heated 100°C for 20 min (dark grey bar), or rhsp70 (0.5µg/ml) (black bar). The results are representative of 2 separate experiments.

### Figure 8

A. Freshly isolated monocytes or B. Monocyte derived DC obtained after 8 days in culture in medium containing GM-CSF and IL-4 alone or C. Monocyte derived DC cultured in GM-CSF/IL-4 containing medium with a cytokine maturation cocktail added from day 6 to 8 were stained for CD14 or CD83 and PE labeled. Cells were also incubated with FITC conjugated BSA or FITC conjugated rhsp70. FITC conjugated rhsp70 bound to CD 14 + monocytes and to immature DC (CD83 low or negative) but not mature DC (CD83 high) None of the cells bound FITC conjugated BSA. Results are representative of 3 separate experiments.

### Detailed description of the invention:

### 1. Hsp70 induces the maturation of monocyte derived dendritic cells when added to immature dendritic cells:

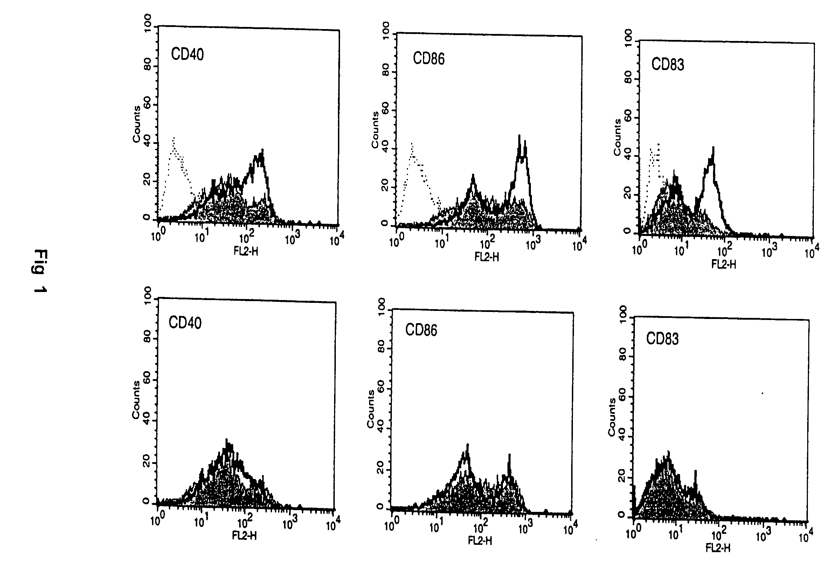

The phenotype of the starting population of plastic adherent mononuclear cells used to generate DC was characterized by flow cytometry. The cells consisted of an average 70% monocytes. Human rhsp70 (0.5 µg/ml) was added to monocyte derived DC after 5 days of culture in GM-CSF and IL-4 containing medium and a FACS analysis was done on day 8. An increase in rhsp70 induced maturation was observed in comparison to control cultures as evidenced by an increase in the expression of CD40, CD86 and CD83 molecules (Fig 1.). No increase in DC maturation was seen in parallel cultures after the addition of heat denatured (100°C, 20 min) human rhsp70 (0.5 mg/ml) (Fig 1).

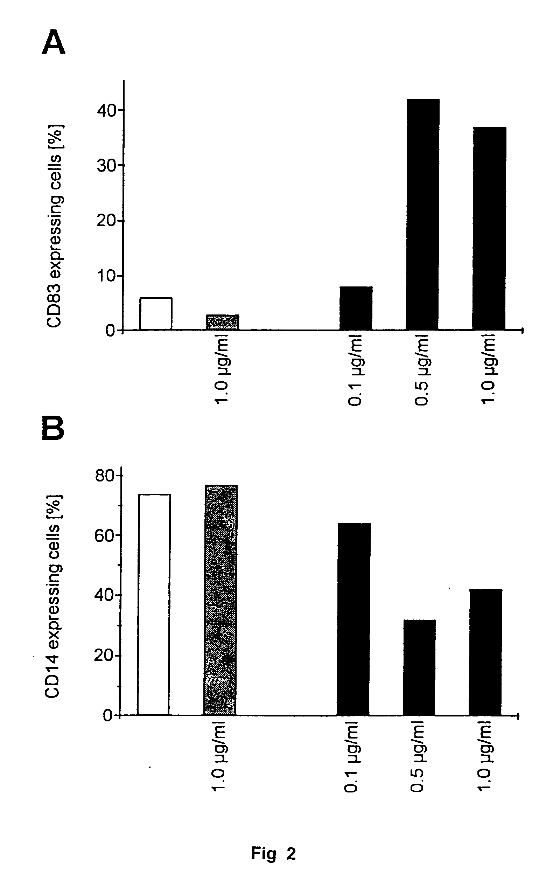

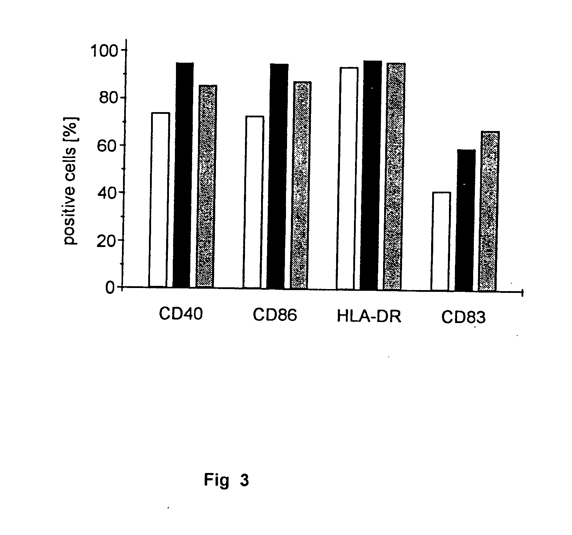

When a comparison was made between the effect of rhsc70 and rhsp70 on DC cell maturation, CD83 and CD14 expression were the same as control values when rhsc 70 was added (Figs. 2A and 2B) whereas the addition of rhsp70 at the same concentration increased CD83 expression (Fig 2A) and decreased CD14 expression (Fig 2B). The addition of polymyxin B (a potent inhibitor of LPS) to cultures had no inhibitory effect on hsp70 induced DC maturation (Fig 3).

### 2. Dendritic cells induced to mature with rhsp70 show an enhanced ability to stimulate specific T cell clones:

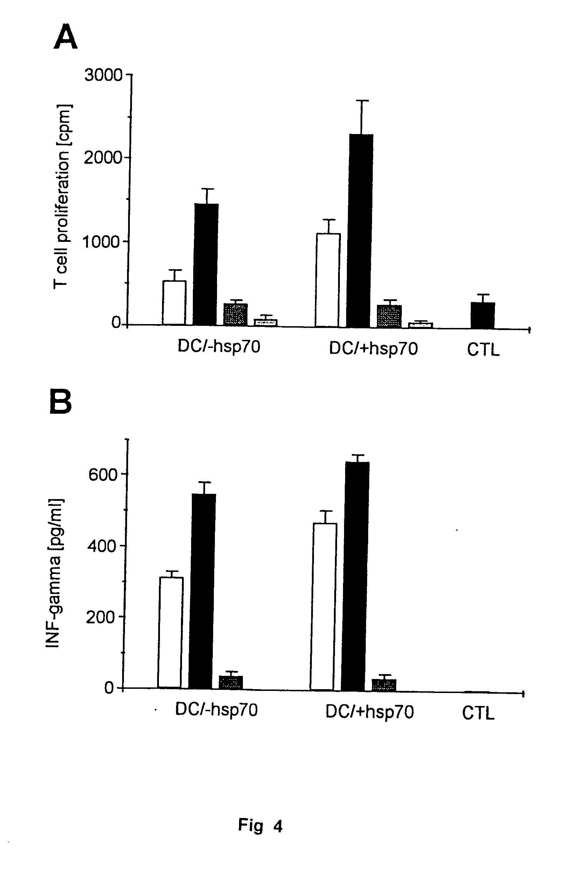

We performed a proliferation assay using HLA-A*0201 restricted T cell clones specific for the tyrosinase 369-377 nonapeptide a known HLA-A*0201 restricted CTL epitope. DC from compatible donors cultured in the presence of rhsp70 (0.5µg/ml) from days 5 to 8 showed an enhanced ability to stimulate tyrosinase specific T cell clones when pulsed with high or low peptide concentrations (1-10 µg/ml) when compared to pulsed DC cultured in GM-CSF and IL-4 only (Fig 4A). In parallel cultures set up to measure IFN-γ production from the CTL clone, the DC cultured in the presence of rhsp70 (0.5µg/ml) from days 5 to 8 and pulsed with tyrosinase peptide also showed an increased ability to stimulate IFN-γ production from tyrosinase specific CTL (Fig 4B). No IFN-γ production was obtained from DC or CTL alone. Very low levels of IFN-γ production were observed in DC /CTL cultures in which the DC had not been pulsed with tyrosinase peptide (Fig 4B).

### 3. Hsp70 reduces the level of DC maturation when added to monocytes at the initiation of culture:

Although rhsp70 could induce DC maturation when added to immature DC it was found that it had the opposite effect when added to monocyte cultures.

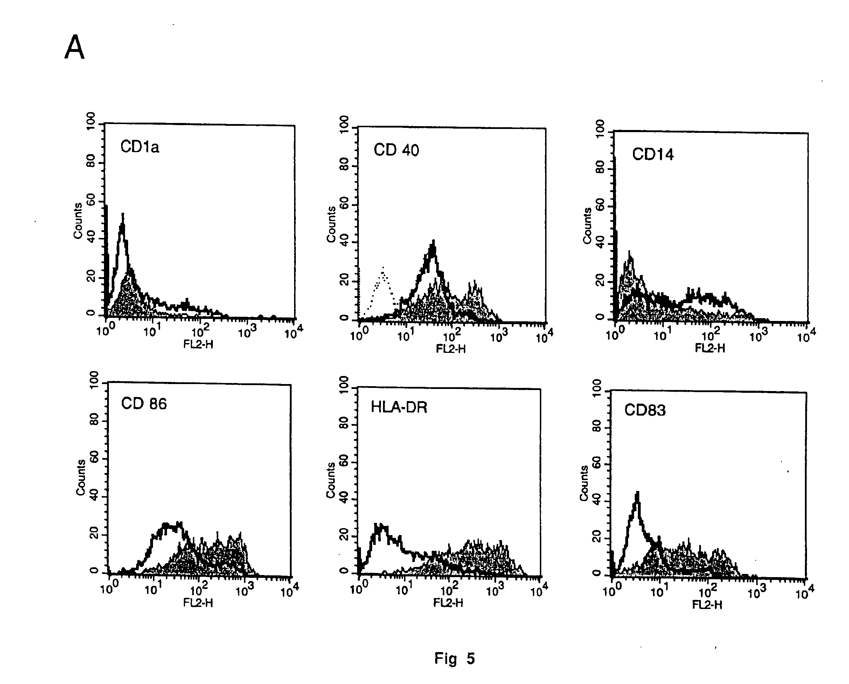

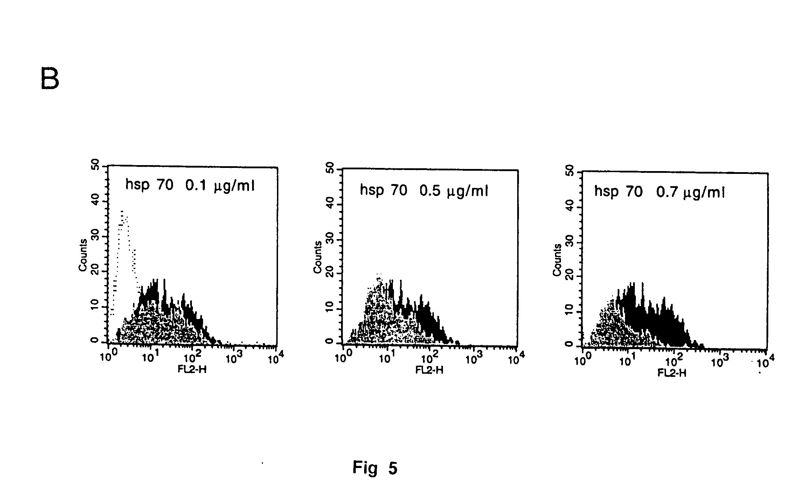

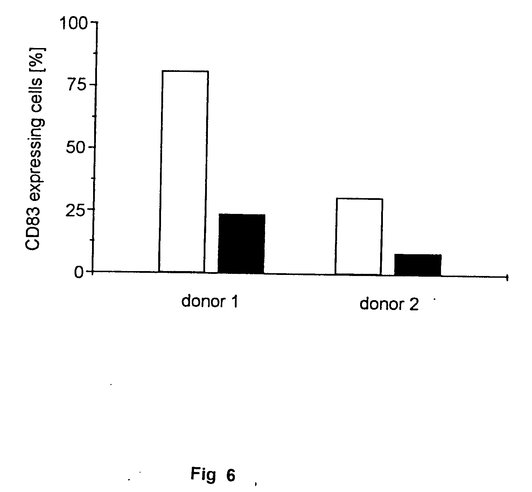

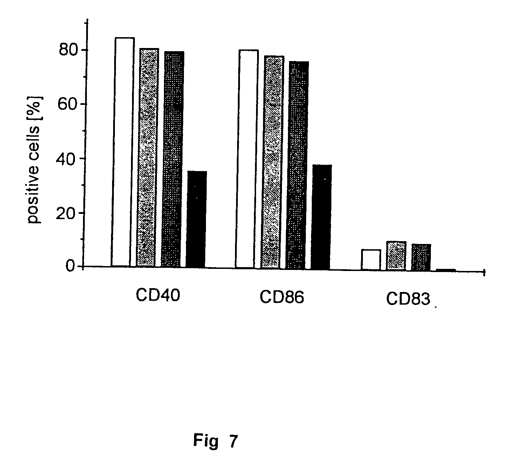

Human rhsp70 (0.1 - 1µg/ml) was added at the same time as GM-CSF and IL-4 to adherent monocytes at the initiation of culture. The DC generated after 8 days showed reduced levels of maturation in comparison to control cultures when rhsp70 was present in the cultures. FACS analysis showed a decrease in the expression of CD1a, CD40, CD83, CD86 and HLA-DR molecules and an increase in the expression of CD14 in comparison to control cultures (Fig 5A). The inhibitory effect was concentration dependent with the maximum effect being found between 0.5 and 0.7µg/ml of rhsp70 (Fig 5B). High or moderate CD83 expression could be found in some control cultures after 8 days but in each case the presence of rhsp70 from day 0 caused a reduction in the number of CD83 positive cells (Fig 6). Heat treated rhsp70 (100°C for 20 min) and rhsc 70 were also added at the initiation of culture at the same concentration as rhsp70 (0.5µg/ml) but had no inhibitory effect on DC generation (Fig 7).

### 4. Monocytes, immature DC and mature DC differ in their ability to bind rhsp70:

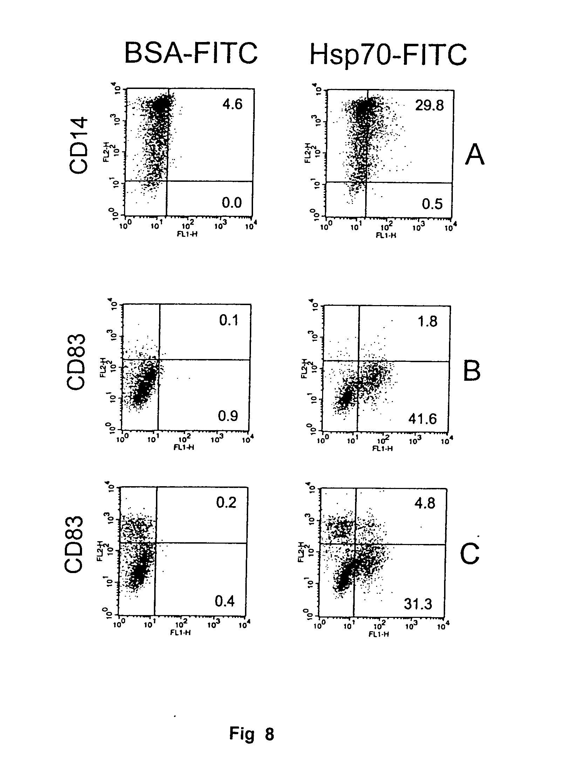

Monocytes obtained after a 2h adherence to plastic were analyzed for their ability to bind rhsp70. A moderate number of CD14+ cells bound rhsp70 (Fig 8A). When monocyte derived DC grown for 8 days in medium containing GM-CSF and IL-4 were incubated with the FITC labeled rhsp70 the immature DC expressing either low levels of CD83 or no CD83 bound rhsp70 to a greater extent (Fig 8B) than the monocytes alone (Fig 8A). When the DC were stimulated to mature by adding a cytokine maturation cocktail (containing IL1-β, IL-6, TNF-α, PGE2) to cultures on day 6 to day 8 the DC expressing high levels of CD83 (mature) showed minimal binding of rhsp70 (Fig 8 C). FITC labeled BSA did not bind to either immature or mature DC populations (Fig 8 B & C).

These results show the specific ability of rhsp70 to induce the maturation of immature (differentiated) DC. However the opposite effect is found when hsp70 is added to monocytes (differentiating precursors) at the same time as GM-CSF and IL-4 in that DC maturation is reduced. These effects were found only with rhsp70 and not with rhsc70 or heat treated rhsp70. We also show that immature DC could bind rhsp70 whereas mature DC could not. Functional studies also revealed that immature DC stimulated with rhsp70 were better able to present peptides to specific T cell clones in comparison to DC cultured in GM-CSF and IL-4 alone.

It has been reported that exogenous hsp70 can bind to the surface CD 14 receptor of human monocytes with subsequent upregulation in the expression of pro-inflammatory cytokines such as TNF-α, IL-6 and IL1-β [11]. A combination of these cytokines plus PGE2 has been used to induce the maturation of immature DC for immunotherapeutic purposes [19]. If monocytes in the presence of GM-CSF and IL-4 could be triggered directly by hsp70 induced cytokines to differentiate into mature DC this as has been suggested [20] would not be the most efficient mechanism for inducing immunity since immature DC need to capture and process antigens. It was found [20] that the presence of hsp70 in tumor cell lysates could target immature DC precursors and maintain the DC population in a more poorly differentiated state. With respect to monocyte precursors as we have shown the presence of hsp70 reduces the maturation of dendritic cells however it may be that in addition to stimulating the production of IL1-β, IL-6 and TNF-α from monocytes [11], hsp 70 stimulates the production of other inflammatory cytokines such as M-CSF which would shift the balance more in the direction of monocytes [21]. Our own results have shown that immature DC can bind and be stimulated to mature by rhsp70. In contrast, mature DC no longer bind rhsp70 which may be due to a down regulation of the receptor for hsp70. Another stress protein, gp96, can induce DC maturation [18] and the binding of gp96 by its receptor, recently characterized as CD91 [22 ] is also down regulated in mature DC [18]. Thus immature DC that can bind specific heat shock proteins such as hsp70 and gp96 are more likely to be able to capture and process antigens whereas mature DC that have lost the ability to bind heat shock proteins would be better at antigen presentation.

Dendritic cells can also deliver Ag directly after incubation with preprocessed synthetic peptide to class I restricted cytotoxic T cells [23]. DC pulsed with tumor derived peptides have been used in immunotherapy trials of certain tumors such as melanoma [24]. It has recently been reported that peptide pulsed mature DC are better than peptide pulsed immature DC in activating CD8+ T cell responses [25]. A tyrosinase peptide derived from melanoma Ags can be presented by DC in association with HLA-A*0201 molecules and stimulates a specific CD8+ T cell response [26]. When we used a CD8+ T cell clone that recognizes a peptide epitope derived from human tyrosinase we found that immature DC treated with rhsp70 were more efficient in presenting the tyrosinase peptide to the specific CTL cell clone.

Thus immature DC's stimulated to mature with rhsp70 and then pulsed with tumor peptides according to the invention are very useful in enhancing a tumor specific immune response.

Since recombinant hsp70 can enhance cytokine production from monocytes [11], and also enhances NK cell proliferation and cytotoxicity whereas hsc70 does not [27], it appears that rhsp70 enhances both specific and innate immune responses. Hsp70 acts as a danger signal that is recognized by both DC and NK cells thus inducing the activation of both the adaptive and innate immune responses and promoting cross talk [28] between the two systems. Induction of hsp70 on tumors in vivo by hyperthermia may also provide a danger signal to the immune system that promotes an anti tumor response in vivo [29].

### Examples:

### 1. Generation of dendritic cells:

Peripheral blood mononuclear cells (PBMC) were prepared from leukapheresis samples by density gradient centrifugation over Ficoll/hypaque (Pharmacia, Biotech, Freiburg, Germany). To obtain CD14+ monocytes, 30 x10⁶ PBMNs were incubated in 75cm² plastic flasks (Nunc, Wiesbaden, Germany) for 2h and the non adherent cells washed off. The adherent cells were then cultured for 8 days in RPMI VLE (Biochrom, Berlin, Germany) supplemented with 2mM glutamine, 100 U/ml pen/strep (all from Life Technologies, Karlsruhe, Germany) and 1% autologous serum. To generate DCs, GM-CSF (500U/ml) (Hölzel Diagnostika, Köln, Germany) and IL-4 (800U/ml) (Biomol, Hamburg, Germany) were added on day 0 and GM-CSF was added again on day 4 of culture.

### 2. Stimulation of monocytes and immature dendritic cells:

Human recombinant hsp70 (0.1-1 µg/ml) (StressGen Biotechnologies, Victoria Canada) was added to monocytes on the same day as the addition of GM-CSF and IL-4, day 0, (i.e. to differentiating precursors) or after the monocytes had been cultured in GM-CSF and IL-4 for 5 days (i.e. to differentiated DC). A FACS analysis of cell surface markers was done on day 8. Control cultures were set up in medium plus GM-CSF and IL-4 alone or with the addition of human recombinant hsc70 (0.5 -1µg/ml) (StressGen Biotechnologies, Victoria, Canada) or human recombinant hsp70 heated (0.5-1µg/ml) (100°C for 20 min) either at the initiation of culture (day 0) or on day 5 of culture. Parallel control cultures containing polymyxin B (0.5µg/ml) (Sigma, Deisenhofen, Germany) were also included.

### 3. FACS analysis:

The antibodies used to assess DC maturation by FACS analysis included CD1a, CD40, CD86 (Pharmingen Hamburg, Germany), CD14 and CD83 (Immunotech, Hamburg Germany) and HLA-DR. The isotype controls used included IgG1, IgG2a and IgG2b (all from Immunotech). Cells were washed in PBS containing 5% FCS. Staining was performed at 4°C for 30 min using mouse mAbs to the markers mentioned above. The cells were then washed and incubated with PE-conjugated goat anti-mouse IgG (Dako, Hamburg, Germany) for 30 min at 4°C. The cells were then washed and resuspended in 500µl of PBS (Life Technologies) plus 5% FCS (Biochrom). All FACS analyses were performed on a FACScan (Becton Dickinson, Mountain View CA) using Cell Quest Software.

### 4. T cell proliferation assay and IFN-γ production:

Monocytes were isolated from an HLA-A*0201 donor as described above. Human DC were generated in GM-CSF and IL-4 containing medium. Hsp70 (0.5µg/ml) was added to immature DC from days 5 to 8 of culture. The cells were then harvested and resuspended at 10,000 DC/ well in 100µl of medium. in 96 well round bottomed plates (Nunc). The cells were then pulsed with tyrosinase 369-377 peptide (1-10µg/ml) for 2h then irradiated. Tyrosinase peptide specific T cells 2 X 10⁴ in 100µl RPMI medium (Biochrom) containing 10% FCS and 100U/ml IL2 (Biomol) were then added to each well. Control wells contained non tyrosinase pulsed DC and CTL or DC alone or CTL alone. Cells were incubated for 72 h at 37°C and 1µCi of ³H thymidine (Amersham Pharmacia Biotech, Freiburg, Germany) was added to the wells for the last 24h of culture. The amount of ³H thymidine incorporated was detected using a microBeta counter (Beckman, Germany).

Parallel cultures were also set up and the supematants (100µl) removed after 24h of culture. The amount of IFN-γ produced was determined using an IFN-γ specific ELISA kit. (cytimmune, Maryland).

### 5. FITC labeling of rhsp70:

Recombinant Hsp70 (Stressgene) and BSA fraction V (Sigma) were incubated with FITC (Sigma) in 0.1 M carbonate-bicarbonate buffer over-night at 4°C with gentle agitation. Free FITC and low molecular reaction by-products were removed by separating the mixture by gel filtration utilizing Sephadex G-25. Fractions containing protein were collected. The number of FITC molecules was estimated to be between 3 to 4 per molecule of protein by comparison of the optical densities at 280, 495, and 490 nm. The conjugated proteins were tested for identity by SDS-PAGE and immuno blotting with the respective specific antibodies against Hsp70 (SPA810, Stressgene), and with anti-FITC mAb (Dako, Hamburg, Germany).

### 6. FACS analysis of binding of FITC labeled rhsp70 to monocytes immature and mature DC:

Monocytes were obtained after a 2h adherence of PBMC to plastic. The non adherent cells were washed off and the adherent cells collected. Immature DC were generated by culturing monocyte precursor cells in GM-CSF and IL-4 containing medium for 8 days as described in section 4.1. Mature DC were also obtained by adding a maturation cocktail containing IL-1β, IL-6, TNF-α and PGE2 [19] to DC on day 6 to day 8. Cells were stained for CD14 and CD83 and PE labeled as described in section 4.3. The cells were then incubated with the FITC-conjugated proteins for 30 min on ice in medium containing 1% autologous serum at a concentration of 10 µg/ml. After washing the cells were fixed with paraformaldehyde and analyzed by flow cytometry. Cells were also labeled with propidium iodide and positive cells were gated out.

### References:

1 **Schlesinger, M. J., Ashburner M. and Tissieres A.** Heat shock: from bacteria to man. Cold Spring Harbour (New.York: Cold Spring Harbour Laboratory Press) 1989. p1-297.
2 **Kiang J.G. and Tsokos G.C.** Heat shock protein 70k Da: Molecular Biology,Biochemistry and Physiology. *Pharmacol. Ther.* 1998. **80:** 183-201.
3 **Vanbuskjrk A., Crump, B.L., Margoliash E. and Pierce S.K** A peptide binding protein having a role in antigen presentation is a member of the HSP70 heat shock family. *J.Exp.Med*.1989. **170:** 1799-1809.
4 **Multhoff G, Botzler C, Wiesnet M,.Eissner G and.Issels R..** CD3- large granular lymphocytes recognize a heat-inducible immunogenic determinant associated with the 72-kd heat shock protein on human sarcoma cells. *Blood* 1995a**86**: 1374-1382.
5 **Multhoff G., Botzler C., Wiesnet M., Müller E., Meier T., Wilmanns W. and Issels R.** A stress-inducible 72kDa heat shock protein (HSP72) is expressed on the surface humantumor but not on normal cells. *Int.J.Cancer.* 1995b.**61**: 272-279.
6 **Botzler C., Issels R. and Multhoff G..** Heat shock protein 72 cell-surface expression on human lung carcinoma cells is associated with an increased sensitivity to lysis mediated by adherent natural killer cells. *Cancer Immunol. Immunother.* 1996. **43:** 226-230.
7 **Botzler C., Schmidt J., Luz A., Jennen L., Issels R. and Multhoff G.** Differential Hsp70 plasma membrane expression on primary human tumors and metastases in mice with severe combined immunodeficiency. *Int.J.Cancer*.1998. **77***:* 942-948.
8 **Multhoff G., Botzler C., Jenne L., Schmidt J.,Ellwart J. and Issels R.** Heat shock protein 72 on tumor cells. A recognition structure for natural killer cells. *J.Immunol.* 1997.**158:** 4341-4350
9 **Chen W., Syldath U., Bellmann K., Burkart V. and Kold H.** Human 60-kDa heat shock protein: A danger signal to the innate immune system.*J.Immunol*. 1999.**162***:* 3212-3219
10 **Todryk S, Melcher A.A., Dalgleish A.G. and Vile R.G.**Heat shock proteins refine the danger theory. *Immunology.2000* **99**:334-337
11 **Asea A., Kreaft S.K., Kurt-Jones E.A., Stevenson M.A., Chen L.B., Finberg R.W., Koo C.G. and Calderwood S.K**...HSP70 stimulates cytokine production through a CD14 dependent pathway, demonstrating a dual role as a chaperone and cytokine..*Nat.Med*..2000. **6** :435-442.
12 **Matzinger P.** Tolerance, danger and the extended family. *Ann.Rev.Immunol*. 1994 **12:** 991-1045.
13 **Medzhitov R. and Janeway C.A.** Innate immunity: The virtues of a nonclonal system of recognition. *Cell* 1997.**91**: 295-298.
14 **Medzhitov R. and Janeway C.** Innate Immunity. *New.Eng.J.Med*.2000. **343:** p338-343.
15 **Banchereau J. and Steinman R.M.** Dendritic cells and the control of immunity. *Nature.* 1999. **392:** p245-252
16 **Colaco C.** Why are dendritic cells central to cancerimmunotherapy ? *Mol. Med. Today* 1999. **5** : 14-17.
17 **Sallusto F. and Lanzavecchia A..** Efficient presentation of soluble antigen by cultured human dendritic cells is maintained by granulocyte/macrophage colon-stimulating factor plusinterleukin 4 and downregulated by tumor necrosis factor alpha. *J.Exp.Med.* 1994. **179:** 1109-1118.
18 **Singh-Jasuja H., Scherer H.U., Hilf. N., Arnold-Schild D., Rammensee H.G., Toes R.E.M. and Schild H.** The heat shock protein gp96 induces maturation of dendritic cells and down-regulation of its receptor.*Eur.J.Immunol 2000*.**30*:*** 2211-2215
19 **Thurner B., Röder C., Dieckmann D., Heuer, M., Kruse M., Glaser A., Keikavoussi P., Kämpgen E., Bender A. and Schuler G.** Generation of large numbers of fully mature and stable dendritic cells from leukapheresis products for clinical application*J.Immunol*. *Methods.* 1999. **223:** 1-15.
20 **Todryk S., Melcher A.A., Hardwick., N., Linardakis E., Bateman A., Colombo M.P., Stoppacciaro. A and. Vile R.G.** Heat shock protein 70 induced during tumor cell killing induces Th1 cytokines and targets immature dendritic cell precursors to enhance antigen uptake. *J.Immunol*.1999. **163:** 1398-1408.
21 **Häusser G., Ludewig B., Gelderblom H.R, Tsunetsugu-Yokota Y., Akagawa K. and Meyerhans A**..Monocyte-derived dendritic cells represent a transient stage of differentiation in the myeloid lineage *Immunobiol.* 1997. **197:** p534-542.
22 **Binder R.J., Han D.K.and Srivastava P.K.** CD91: a receptor for heat shock protein gp96. *Nature Immunology* 2000. **1** : p151-155.
23 **Mavordomo J I., Zorina T., Storkus W J., Zitogel L., Celluzzi C., Falo L D., Melief C J., Ildstad S T., Kast M W., Deleo A A. and Lotze M T.** Bone marrow-derived dendritic cells pulsed with synthetic tumour peptides elicit protective and therapeutic antitumour immunity. *Nat.Med*.1995.**1:**1297-1302.24 **Nestle F.O, Alijagic S., Gillet M., Yuansheng S., Grabbe S., Dummer R., Burg G. and Schadendorf D.** Vaccination of melanoma patients with peptide or tumor lysate-pulsed dendritic cells. *Nat.Med.*1998 **4**.328-332.
25 **Bullock T.N.J., Colella T A. and Engelhard V.H.** The density of peptides displayed by dendritic cells affects immune responses to human tyrosinase and gp100 in HLA-A2 transgenic mice. *J.Immunol.* 2000.**164:**2354-2361.
26 **Visseren M.J.W., van Elsas A., van der Voort E.J.H., Ressing M.E., Kast M W., Schrier P.L. and Melief C.J.M. CTL** specific for the tyrosinase autoantigen can be induced from healthy donor blood to lyse melanoma cells. *J.Immunol.* 1995.**154:** 3991-3998.
**27 Multhoff G., Mizzen L., Winchester C.C.,Milner C.M., Wenk S., Eissner G., Kampinga H H., Laumbacher B. and Johnson. J.** Heat shock protein 70 (Hsp 70) stimulates proliferation and cytolytic activity of natural killer cells. *Exper.Hematol.* 1999. **27*:*** 1627-1636.
28 **Fernandez N.C., Lozier A., Flament C., Ricciardi-Castagnoli P., Bellet D., Suter M., Perricaudet M., Tursz T., Maraskovsky E. and Zitvogel** L. cells directly trigger NK cell functions: Cross-talk relevant in innate anti-tumor immune responses in vivo. *Nat.Med.* 1999.**5**: 405-411.
29 **Fuller K.J., Issels R.D., Slosman D.O., Guillet J-G., Soussi T. and Polla B.S.** Cancer and the heat shock response. *Eur.J.Cancer* 1994. **306:** 1884-1891.

## Claims

1. An ex vivo method for inducing the TNF-α free differentiation of immature dendritic cells into mature dendritic cells, said method comprising contacting the immature dendritic cells with an effective amount of heat shock proteins of the hsp70 family or a biologically active part thereof.

2. The method of claim 1, wherein the biologically active part is the C-terminal domain of hsp 70.

3. The method of claim 1 - 2, wherein said immature dendritic cells are generated by culturing monocytes in an induction medium containing granulocyte/macrophage-colony stimulating factor ("GM-CSF") and interleukin-4 ("IL-4").

4. An ex vivo method for generating mature dendritic cells, said method comprising:
(a) inducing the differentiation of immature dendritic cells into mature dendritic cells, by contacting the immature dendritic cells with an effective amount of heat shock proteins of the hsp70 family or a biologically active part thereof free of TNF-α; and
(b) recovering said mature dendritic cells.

5. The method of claim 4, wherein the biologically active part is the C-terminal domain of hsp 70.

6. The method of claim 4 or 5, wherein said immature dendritic cells are generated by culturing monocytes in an induction medium containing granulocyte/macrophage-colony stimulating factor ("GM-CSF") and interleukin-4 ("IL-4").

7. The method of claims 3 and 6, wherein the monocytes are human blood cell monocytes.

8. The method of claim 7, wherein the monocytes are plastic-adherent monocytes.

9. The method of any one of claims 1 - 8, wherein the mature dendritic cells are further pulsed with an antigenic agent.

10. The method of any one of claims 1 - 9, wherein hsp 70 is recombinant (rhsp 70).

11. The method of claim 10, comprising maintaining the rhsp70 concentration in the culture medium in the range of about 0.1 - 1.0 µg/ml.

12. The method of claim 11, wherein the concentration of rhsp70 is about 0.5 µg/ml.

13. Mature dendritic cells obtainable by the methods of any one of claims 4 - 6.

14. A TNF-α free therapeutic composition for inducing the maturation of immature dendritic cells comprising, as the only active maturation agent, an effective amount of heat shock proteins of the hsp70 family or a biologically active part thereof, in combination with a pharmaceutically acceptable carrier.

15. The therapeutic composition of claim 14, comprising rhsp70.

16. Therapeutic composition comprising the mature dendritic cells of claim 13 in combination with a pharmaceutically acceptable carrier.

17. The therapeutic composition of any one of claims 14 - 16 that is a vaccine.

18. Use of the composition of any one of claims 14 - 17 in the immunotherapy of neoplastic diseases.

19. Use of the composition of any one of claims 14 - 17 for inducing the TNF-α free maturation of immature dendritic cells
